Europäisches Patentamt

European Patent Office

Office européen des brevets

(19)

(11) Numéro de publication: **0 207 846 B1**

(12) **FASCICULE DE BREVET EUROPÉEN**

(45) Date de publication de fascicule du brevet:
04.09.91

(51) Int. Cl.5: **C07K 5/06, //C12P21/04**

(21) Numéro de dépôt: **86401338.8**

(22) Date de dépôt: **18.06.86**

(54) **Procédé pour la séparation et l'isolement du nosiheptide.**

(30) Priorité: **19.06.85 JP 133552/85**

(43) Date de publication de la demande:
**07.01.87 Bulletin 87/02**

(45) Mention de la délivrance du brevet:
**04.09.91 Bulletin 91/36**

(84) Etats contractants désignés:
**AT BE CH DE FR GB IT LI LU NL SE**

(56) Documents cités:
**EP-A- 0 073 329**
**EP-A- 0 133 079**

CHEMICAL ABSTRACTS, vol. 103, 1985, page
580, résumé no. 194965g, Columbus, Ohio,
US; & JP-A-60 120 993 (MITSUBISHI CHEMI-
CAL INDUSTRIES CO., LTD) 28-06-1985

CHEMICAL ABSTRACTS, vol. 105, 1986, page
515, résumé no. 96051c, Columbus, Ohio, US;
& JP-A-61 25 495 (MITSUBISHI CHEMICAL
INDUSTRIES CO., LTD) 04-02-1986

(73) Titulaire: **RHONE-POULENC SANTE**
**20, avenue Raymond Aron**
**F-92160 Antony(FR)**

(72) Inventeur: **Morioka, Satoshi MITSUBISHI CHE-**
**MICAL INDUSTR. LTD.**
**Central Research No. 1000, Kamoshida-cho**
**Midori-ku Yokohama-shi Kanagawa(JP)**
Inventeur: **Shida, Makoto MITSUBISHI CHEMI-**
**CAL INDUSTR. LTD.**
**Central Research No. 1000, Kamoshida-cho**
**Midori-ku Yokohama-shi Kanagawa(JP)**

(74) Mandataire: **Le Pennec, Magali et al**
**RHONE-POULENC SANTE, Service Brevets,**
**20 Avenue Raymond Aron**
**F-92165 Antony Cédex(FR)**

## Description

La présente invention concerne un procédé pour la séparation et l'isolement du nosiheptide, qui est un antibiotique, et plus particulièrement l'invention a pour objet un procédé de séparation et d'isolement du nosiheptide à partir d'un moût de fermentation.

Le nosiheptide, appelé également 9671 RP est un antibiotique qui est produit par culture d'une souche appartenant au genre Streptomyces et qui est utile comme adjuvant d'alimentation animale.

Il est connu de préparer le nosiheptide par culture d'une souche productrice de nosiheptide appartenant au genre Streptomyces (brevet japonais publié sous le numéro 880/1965 ou brevet américain US 3 155 581). Pour séparer et isoler le nosiheptide à partir d'un milieu de culture, il a été proposé une méthode consistant à utiliser un éther cyclique comme solvant d'extraction (demande de brevet japonais JP 121273/1973 ou demande de brevet européen EP 133079) ou à ajouter un sel minéral après le traitement par un solvant, mais ces méthodes ne sont pas satisfaisantes.

Généralement, la séparation et l'isolement d'un antibiotique à partir d'un milieu de culture sont effectués en mélangeant le milieu de culture avec un solvant, en séparant le mycelium insoluble dans le solvant, en éliminant le solvant par distillation par chauffage ou sous pression réduite puis en séparant les particules de l'antibiotique précipité par filtration ou centrifugation. Ce procédé ne peut pas être facilement mis en oeuvre et la pureté du nosiheptide diminue puisque les particules sont très petites.

Devant la situation ci-dessus, l'objet de la présente invention est de fournir une méthode qui, permet la séparation et l'isolement du nosiheptide avec une grande pureté et des rendements élevés à partir d'un milieu de culture contenant le nosiheptide en améliorant les caractéristiques de sédimentation et de filtration des particules du nosiheptide.

Le but est atteint en ajoutant et en mélangeant de l'eau ou de l'eau contenant un acide avec une solution de nosiheptide, issue d'un procédé pour la séparation et l'isolement du nosiheptide et qui est composée du mélange du milieu de culture d'une souche productrice de nosiheptide appartenant au genre Streptomyces avec un solvant qui est soluble dans l'eau, et en isolant le nosiheptide de la solution après séparation d'une fraction insoluble dans le solvant ayant le mycélium comme constituant principal.

Comme souches productrices de nosiheptide sont connus Streptomyces actuosus (NRRL 2954; ATCC 25421) et ses mutants appartenant au genre Streptomyces. Le milieu de culture contenant le nosiheptide est obtenu en cultivant une telle souche selon le procédé décrit dans le brevet japonais publié sous le numéro 880/1965 ou le brevet américain US 3 155 581). Le milieu de fermentation contient des hydrates de carbone, des sels minéraux, qui proviennent de la composition du milieu de culture, le mycélium et le nosiheptide. Le nosiheptide s'accumule à la surface du mycélium principalement composé d'actinomycetes ayant une structure de surface compliquée.

Le procédé selon l'invention est caractérisé par le mélange du milieu de cultre (moût) avec un solvant. Les solvants comprennent les cétones, les alcools et les dérivés furanniques qui sont solubles dans l'eau et de préférence l'acétone, la méthyléthylcétone, les alcools aliphatiques ayant moins de 4 atomes de carbone et le tétrahydrofuranne.

Comme milieu de culture convenant pour le traitement par un solvant peut être utilisé soit un moût ayant subi un traitement de deshydratation par un moyen mécanique, tel que la centrifugation ou la filtration, soit un moût brut n'ayant pas subi de traitement de deshydratation. Il est économiquement avantageux d'utiliser un moût brut.

La quantité de solvant utilisée est variable selon la nature du solvant et la quantité d'eau et de nosiheptide dans le milieu de fermentation. Par exemple, lorsqu'on utilise le tétrahydrofuranne, l'acétone et l'éthanol, la quantité est généralement comprise entre 0,5 et 5 fois en volume, de préférence 1,5 à 2,5 fois en volume par rapport au volume du milieu de culture.

Le traitement par le solvant est généralement réalisé en utilisant un réservoir agité ayant une trappe dans le fond afin de pouvoir éliminer la matière insoluble. Le temps de traitement est généralement compris entre 0,5 et 3 heures, de préférence entre 1 et 2 heures, et la température est habituellement comprise entre 5 et 80° C et de préférence entre la température ambiante et 60° C.

Le pH du milieu de culture utilisé dans le traitement varie selon les conditions de la culture. Il est préférable d'ajuster le pH entre 3 et 10 et plus particulièrement entre 5 et 9 au moment du traitement.

Après le traitement par un solvant, une fraction insoluble constituée essentiellement du mycélium est séparée du milieu de culture et du solvant pour obtenir une solution de nosiheptide.

Le procédé selon la présente invention est caractérisé par l'addition d'eau ou d'eau contenant un acide à la solution contenant le nosiheptide obtenue en vue de séparer le nosiheptide. La quantité d'eau ou d'eau contenant un acide ajoutée représente de 0,3 à 2 fois le volume de la solution de nosiheptide et de préférence 0,4 à 1 fois. La séparation ne s'effectue pas convenablement si l'on ajoute une petite quantité

2

d'eau et en revanche, si l'on en ajoute trop, les caractéristiques de filtration deviennent mauvaises.

Comme acide, on utilise seul ou en mélange, un acide minéral tel que l'acide sulfurique, l'acide chlorhydrique ou l'acide nitrique ou un acide organique tel que l'acide acétique, en quantité telle que le pH de la solution soit compris entre 3 et 7 et de préférence entre 5 et 6 quant on ajoute de l'eau contenant un acide à la solution. Quand le pH est élevé, le taux de précipitation et la pureté chimique du nosiheptide deviennent mauvais.

L'addition d'eau ou d'eau contenant un acide doit être faite progressivement en mélangeant et en agitant. Généralement l'addition d'eau ou d'eau contenant un acide est effectuée en 0,1 à 5 heutes et de préférence en 0,5 à 3 heures.

Ainsi la manière dont on ajoute l'eau ou l'eau contenant un acide influence grandement la taille des particules de nosiheptide et sa pureté. Par exemple, en ajoutant 4 m3 d'eau à 6 m3 de solution contenant le nosiheptide dans un mélangeur ayant un volume utile de 10 m3, la vitesse d'addition étant rapide (10 à 30 minutes soit 0,4 à 0,13 m3 / minute), on obtient des particules inférieures à 0,14 microns et dont la pureté est comprise entre 85 et 90%; en revanche en ajoutant progressivement et en continu (0,5 à 5 heures soit 0,13 à 0,013 m3/ minute) on obtient des particules de 1 à 2 microns et dont la pureté est de 95 à 98%.

Cette opération est habituellement utilisée par séparer des cristaux, cependant dans le cas de produits amorphes comme le nosiheptide elle n'a jamais été mise en oeuvre. L'isolement du nosiheptide qui se sépare de la solution est facilement réalisé par centrifugation ou filtration.

Le procédé de la présente invention qui consiste à ajouter de l'eau ou de l'eau contenant un acide en une période de 0,1 à 5 heures et de préférence de 0,5 à 3 heures améliore les propriétés de sédimentation et de filtration des particules de nosiheptide, ce qui permet une séparation et un isolement faciles.

Le nosiheptide ainsi obtenu contient parfois de faibles quantités de métaux. Si nécessaire, pour éliminer les métaux, le nosiheptide doit être dissous dans un solvant convenable et être traité par du charbon activé, de la silice ou par une résine échangeuse d'ions.

Les exemples suivants, donnés à titre non limitatif, illustrent la présente invention.

## EXEMPLE 1

Dans un mélangeur avec une trappe dans le fond on charge 100 parties en volume d'un milieu de culture (moût) obtenu par culture de Streptomyces actuosus et 200 parties en volume de tétrahydrofuranne. On agite pendant 1 heure à 60°C. Le mycélium est alors séparé des extraits par centrifugation. Le volume des extraits est de 260 parties en volume.

La composition en poids du milieu de culture est la suivante :

| | |
|---|---|
| mycélium | : 7,5% |
| hydrates de carbone | : 2,0% |
| sels minéraux | : 0,5% |
| nosiheptide | : 0,5% |
| eau | :89,5% |

Le pH est de 5,6.

L'analyse par chromatographie en phase liquide montre que les extraits contiennent 0,187% en poids de nosiheptide (rendement 90,0% en poids).

On ajoute alors en 1 heure 150 parties en volume d'eau contenant 0,2 parties en volume d'acide sulfurique concentré dans le mélangeur contenant 200 parties en volume de la solution ci-dessus, puis on filtre le mélange pour séparer le nosiheptide.

D'après l'analyse par chromatographie en phase liquide, la concentration de nosiheptide dans le filtrat est de 0,005% en poids.

La pureté du nosiheptide ainsi isolé est de 98% et le rendement est de 95%.

## EXEMPLE 2

Dans un mélangeur identique à celui décrit dans l'exemple 1, on charge 100 parties en volume d'un milieu de culture (moût) et on ajuste le pH à 8,0 par addition d'une solution de soude à 10% en poids. Puis on ajoute 200 parties en volume d'acétone (au lieu de tétrahydrofuranne). On traite comme dans l'exemple 1. Le volume de la solution contenant le nosiheptide est de 240 parties. L'analyse par chromatographie en phase liquide montre que la solution contient 0,193% en poids de nosiheptide et que le taux de séparation est de 85%.

On ajoute alors en 1 heure, régulièrement en agitant, 140 parties en volume d'eau contenant O,2 parties en volume d'acide sulfurique concentré dans le mélangeur contenant 200 parties en volume de la solution ci-dessus. Puis le mélange est filtré pour séparer le nosiheptide qui présente les mêmes caractéristiques que celui obtenu à l'exemple 1.

La concentration en nosiheptide dans le filtrat est de 0,008%.

La pureté du nosiheptide isolé est de 98% et le rendement est de 92,5%.

EXEMPLE 3

Dans un mélangeur contenant 200 parties en volume d'une solution contenant du nosiheptide qui est obtenue en mélangeant 100 parties en volume de moût avec 200 parties en volume d'acétone puis en séparant l'insoluble comme dans l'exemple 2, on ajoute en 1 heure régulièrement et en agitant, 300 parties en volume d'eau. Le nosiheptide est séparé de la même manière que dans l'exemple 1.

La concentration du nosiheptide dans le filtrat est de 0,007%.

La pureté du nosiheptide isolé est de 97% et le rendement est de 91%.

EXEMPLE COMPARATIF 1

Dans le procédé de l'exemple 1, le milieu de culture et le solvant sont mélangés et agités puis le mélange est chauffé pour distiller le tétrahydrofuranne et précipiter le nosiheptide. Les particules de nosiheptide ainsi séparées ne peuvent pas être séparées par filtration car elles forment une émulsion non filtrable. Les particules sont séparées par centrifugation (5000 g; 20 minutes) et le précipité est lavé.

La pureté du nosiheptide est de 70,0% et le rendement est de 80%.

L'invention permet de précipiter le nosiheptide ayant une grande taille de particules et de bonnes propriétés de filtration à partir d'un milieu de fermentation obtenu par culture d'une souche productrice de nosiheptide et d'isoler le nosiheptide sous forme pure et avec un rendement élevé par une méthode simple.

## Revendications

1. Dans un procédé pour la séparation et l'isolement du nosiheptide qui consiste à mélanger un milieu de culture, obtenu par culture d'une souche productrice de nosiheptide appartenant au genre Streptomyces, avec un solvant qui est soluble dans l'eau puis à séparer du nosiheptide à partir de la solution obtenue après séparation d'une fraction insoluble dans le solvant ayant le mycélium comme constituant essentiel, le perfectionnement consiste à ajouter de l'eau ou de l'eau contenant un acide dans la solution contenant le nosiheptide afin de précipiter le nosiheptide et de séparer le nosiheptide ainsi précipité.

## Claims

1. In a process for the separation and isolation of nosiheptide, which consists in mixing a culture medium, obtained by culture of a strain which produces nosiheptide and belongs to the genus Streptomyces, with a solvent which is water-soluble, and then separating the nosiheptide from the solution obtained after having separated off a fraction which is insoluble in the solvent and has the mycelium as the essential constituent, the improvement consists in adding water, or water containing an acid, to the solution containing the nosiheptide in order to precipitate the nosiheptide and to separate off the thus precipitated nosiheptide.

## Patentansprüche

1. In einem Verfahren zur Abtrennung und Isolierung von Nosiheptid, das darin besteht, daß man ein durch Züchtung eines Nosiheptid produziereiden Stammes aus der Gattung Streptomyces erhaltenes Kulturmilieu mit einem wasserlöslichen Lösungsmittel mischt, dann Nosiheptid aus der erlhaltenen Lösung nach Abtrennung einer in dem Lösungsmittel unlöslichen Fraktion, die das Mycel als Hauptbestandteil hat, abtrennt, besteht die Verbesserung darin, daß man der das Nosiheptid enthaltenden Lösung Wasser oder eine Säure enthaltendes Wasser zusetzt, um das Nosiheptid zu fällen, und das so gefällte Nosiheptid abtrennt.